# EUROPEAN PATENT SPECIFICATION

(11) **EP 4 436 582 B1**
(45) Date of publication and mention of the grant of the patent: **02.04.2025**
(21) Application number: 22847278.3
(22) Date of filing: 08.12.2022
(51) Int. Cl.: A61K 31/565, A61P 43/00

(54) **2-METHOXYESTRADIOL FOR USE IN THE TREATMENT OF MASTOCYTOSIS**
2-METHOXYESTRADIOL ZUR VERWENDUNG BEI DER BEHANDLUNG VON MASTOZYTOSE
2-MÉTHOXYESTRADIOL POUR L'UTILISATION DANS LE TRAITEMENT DE LA MASTOCYTOSE

(30) Priority: 08.12.2021 PL 43978021
(43) Date of publication of application: 02.10.2024
(73) Proprietor: Gdanski Uniwersytet Medyczny, 80-210 Gdansk (PL)
(72) Inventor: GÓRSKA-PONIKOWSKA, Magdalena, 80-442 Gdansk (PL); PELIKANT-MALECKA, Iwona, 80-402 Gdansk (PL); BASTIAN, Paulina, 80-410 Gdansk (PL); NIEDOSZYTKO, Marek, 80-809 Gdansk (PL); NEDOSZYTKO, Boguslaw, 80-336 Gdansk (PL); KALINOWSKI, Leszek, 80-298 Gdansk (PL); WOZNIAK, Micha, 81-881 Sopot (PL); LANGE, Magdalena, 81-862 Sopot (PL); GRUCHALA-NIEDOSZYTKO, Marta, 80-809 Gdansk (PL); NOWICKI, Roman, 81-784 Sopot (PL); GÓRSKA, Aleksandra, 80-171 Gdansk (PL)
(74) Representative: JWP Patent & Trademark Attorneys
(86) International application number: PCT/PL2022/050091
(87) International publication number: WO 2023/106947

(56) References cited:
- WO-A1-2018/050801
- WO-A1-2019/152719
- WO-A1-97/42958
- WO-A2-2007/010014
- US-A1- 2021 077 503
- PETER VALENT ET AL: "Diagnosis and treatment of systemic mastocytosis: state of the art", BRITISH JOURNAL OF HAEMATOLOGY, JOHN WILEY, HOBOKEN, USA, vol. 122, no. 5, 21 August 2003 (2003-08-21), pages 695 - 717, XP071161080, ISSN: 0007-1048, DOI: 10.1046/J.1365-2141.2003.04575.X
- SIBONGA J. D. ET AL: "Dose-Response Effects of 2-Methoxyestradiol on Estrogen Target Tissues in the Ovariectomized Rat", ENDOCRINOLOGY, vol. 144, no. 3, 1 March 2003 (2003-03-01), US, pages 785 - 792, XP093022698, ISSN: 0013-7227, Retrieved from the Internet <URL:http://academic.oup.com/endo/article-pdf/144/3/785/9015451/endo0785.pdf> DOI: 10.1210/en.2002-220632

## Description

### Field of the invention

The invention concerns 2-methoxyestradiol for use in the treatment of mastocytosis.

### State of the art

Mastocytosis is a heterogenic group of diseases related to pathological accumulation of mastocytes (mast cells, hereinafter referred to as MC or MCs) in various organs, most frequently in bone marrow and skin. The main molecular cause of the disease is somatic mutation pD816V of the c-KIT gene coding the membrane receptor for the stem cell growth factor (stem cell factor, hereinafter referred to as SCF). The mutation makes the MC activation independent of SCF, as well as makes the cells oversensitive to the action of stimulating factors and inhibits their apoptosis process. The pD816V mutation is observed in more than 90% of adult patients suffering from a systemic form of mastocytosis, and in approximately 50% of sick children.

Neoplastic MCs which infiltrate tissues in the course of this disease demonstrate atypical morphology and changed immunophenotype (presence of the CD4 and CD25 antigens on the surface), where the features constitute important diagnostic criteria. Mastocytosis is characterised by a broad spectrum of clinical forms related to the MC's release of inflammatory reaction mediators, proteolytic enzymes which degrade tissues, growth factors, cytokines, and chemokines [1-3]. The skin symptoms include maculopapular rash with the Darier's symptom typical for the disease, itching, and the formation of blisters. Mastocytosis is also related to the risk of occurrence of anaphylaxis, osteoporosis, and aggressive symptoms related to the impairment of internal organ functions [4-6].

Described in the patent applications submitted to date were different ways of inhibiting the activity of mast cells (mastocytes) and treating diseases triggered by their activation, none of them, however, concerns the action of steroid hormone derivatives on those cells.

Disclosed in international invention application WO2014188423A1 are methods of inhibiting activation of a mast cell and/or treating the induced disease by contacting a neoplastic mast cell with an effective amount of a multivalent compound which binds and activates membrane protein, i.e. the Siglec-7 lectin (Sialic acid-binding Ig-like lectin 7), thus inhibiting activation of the neoplastic mast cell.

Patent PL201879B has disclosed that hybrid protein binds to (i) mast cells and/or basophils and/or is absorbed thereby and (ii) binds to a protease which splits the proteins of the secretory apparatus of stem cells or basophils. Hybrid protein is composed of IgE or its fragment Fc or an antibody against the IgE receptor of the mast cells and/or basophils, as well as of MCD (inactive but peptide-binding) and proteases. The protein according to the patent application may be used to produce a medicament blocking mast cell degranulation.

Disclosed in patent EP1410802B1 is a fusion protein which inhibits growth of mastocytes and induces their apoptosis. Fusion protein contains protein transduction domain (PTD) and a variant of the microphthalmia-associated transcription factor MITF. Reduction of the number of mastocytes was evidenced with reduced level of histamine and reduced activity of an MC enzyme, chymase.

Described in patent EP95909451B1 is a new medicament blocking the activity of mastocytes and being a heterocyclic H1 histamine receptor. The preparation may be potentially used in induced diseases caused by mastocyte degranulation, such as irritable bowel syndrome, abdominal migraine, food allergy, and other diseases related to mastocyte activation. PETER VALENT ET AL: "Diagnosis and treatment of systemic mastocytosis: state of the art", BRITISH JOURNAL OF HAEMATOLOGY, JOHN WILEY, HOBOKEN, USA, vol. 122, no. 5, 21 August 2003, pages 695-717, reviews the diagnosis and treatment of systemic mastocytosis.

A still unexplained problem concerning the pathogenesis of mastocytosis is the clinically observed regression, sometimes full, particularly in puberty, occurring in most sick children. In some of the adult patients, on the other hand, suffering from the chronic form of the disease, it transforms into an aggressive form (agressive systemic mastocytosis - ASM).

Although the tendency to spontaneous regression of the disease observed in children around puberty might suggest an impact of sex hormones, no research team has so far demonstrated or proved what hormone might potentially affect the process.

In the early stages of research on mastocytosis pathogenesis there were no appropriate in vitro cell models available for the disease.

A solution to the problem came with isolation of two long-lived human MC cell lines: HMC 1.1 and HMC 1.2 from a patient with mast cell leukemia, and experimental introduction of two ROSA cell lines. The ROSA lines were obtained following transformation of the MCs isolated from umbilical blood using lentivirus containing the p.D816V KIT mutation in its genome. The lines enabled an effective search for candidate medicaments to treat and monitor medical conditions, such as allergy, inflammation, autoimmunological disease, or mastocytosis.

### Characteristics of the HMC and ROSA lines

The difference between the HMC lines comes down to the different KIT gene mutations contained therein. The HMC-1.1 line contains the p.V560G KIT mutation in its cells, while the HMC-1.2 line is characterized by the presence of two KIT gene mutations: p.V560G and p.D816V.

The ROSA KIT pD816V line contains the p.D816V mutation, while the ROSA KIT WT line contains no mutation of the KIT gene [21-23]. In addition, information on the ROSA line was disclosed in patent EP2773749B, and cell lines were deposited with CNCM (French National Collection of Cultures of Microorganisms) on 02/11/2011 under numbers: CNCM- 1-4551 ROSA-KIT WT; CNCM-1-4552 ROSA KIT D816V; CNCM 1-4553 ROSA KIT Delta 417-419 insY .

The authors of the invention conducted a number of research studies intended to develop an effective mastocytosis therapy based on hormonal preparations using the cell lines described above. Unexpectedly it turned out that one of the female sex hormones is a promising candidate to be used as a medicament in mastocytosis therapy.

### Detailed description of the invention

The invention concerns 2-methoxyestradiol for use in the treatment of mastocytosis, especially its skin and/or systemic form.

2-methoxyestradiol for use according to the invention is an agent carrying selective cytotoxic effects on neoplastic mastocytes containing the KIT gene mutation.

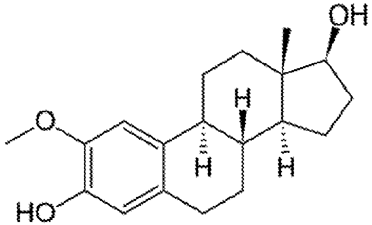

2-methoxyestradiol (hereainafter referred to as 2-ME) is a physiologically-occurring compound; it is a metabolite of 17β-estradiol (E2) belonging to estrogens, female sex hormones [8]. In addition, it was proved that 2-ME is an effective anticancer agent [9].

2-ME, available under the commercial name of Panzem, is in advanced phases of clinical research on the treatment of numerous malignant cancers, including colon cancer, breast cancer, lung cancer, or osteosarcoma [10-13].

The molecular mechanism of action of 2-ME has not yet been fully understood, however it is known to generate reactive forms of oxygen and nitrogen, thus leading to the nitro-oxidative stress which causes cell apoptosis [8].

The major anti-proliferation mechanisms include inhibition of the microtubule formation dynamics, inhibition of neoangiogenesis, and regulation of external and internal apoptotic pathways [14].

It was proved that 2-ME selectively induces neuronal nitric oxide synthase (nNOS) in the 143B osteosarcoma cell line in pharmacological and physiological concentrations. 2-ME increases the presence of nNOS in the nucleus, damaging the DNA by way of the nitro-oxidative stress, which in turn stops the cell cycle and causes apoptosis of the osteosarcoma cells [15-20].

Induction of nNOS in physiological concentrations implies a hypothesis that 2-ME in human organism is not only an active cell metabolite, but also an independently operating hormone [16].

In the conducted research it was observed that 2-ME was selectively cytotoxic to neoplastic MCs, specifically to those which contained KIT gene mutations (lines HMC 1.1, HMC 1.2, ROSA KIT D816V), but did not demonstrate any such activity with respect to regular MCs (line ROSA KIT WT).

It was proved that application of 2-ME in the concentration of 1 µM for 48 hours reduced the viability of the cells of the HMC 1.1 line (containing the p.V560G mutation) down to 71%, whereas at the 2-ME concentration at the level of 5 µM cell viability dropped to 63%, and at the 2-ME concentration of 10 µM to 67%. In the case of the HMC 1.2 cell line (with mutations p.V560G and p. D816V) the number of live cells following the application of 2-ME for 48 hours dropped to the following levels, respectively: 78% for the 2-ME concentration of 1 µM, up to 76% for the concentration of 5 µM, and up to 70% for the concentration of 10 µM.

In the case of the research conducted on the ROSA line cells, the effects in terms of reduced viability of the mastocyte cells treated with 2-ME proved even more spectacular. The ROSA KIT D816V line (containing mutation p. KIT D816V) proved the most susceptible to 2-ME, since following the treatment of those cells with 2-ME at the concentration of 1 µM their viability dropped as much as to 56%. When higher concentrations were applied, i.e. 5 and 10 µM, the number of live cells dropped to 55% and 51%, respectively. In the case of the ROSA KIT WT line (without KIT gene mutation), the 2-ME concentration of 1 µM inhibited the cell growth by the mere 5%, at the concentration of 5 µM by 12%, and at the concentration of 10 µM by 14%.

The obtained results of the research are of momentous importance for understanding the pathogenesis of mastocytosis, particularly in children, and explain the phenomenon of disease regression in puberty, they also suggest a potential possibility to develop new therapies of the skin disease and systemic disease using 2-ME.

In addition, the results of the research indicate that the ROSA KIT D816V and ROSA KIT WT cell lines are very good models for testing effects of active agents on mastocytes.

### Figure description

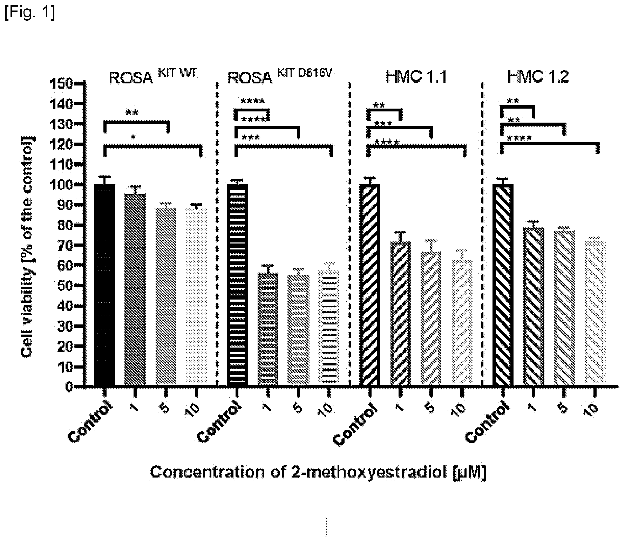

[Fig1] presents the effects attained for various 2-ME (2-methoxyestradiol) concentrations applied for 48 hours on the viability of cells of the HMC 1.1, HMC 1.2, ROSA KIT D816V, ROSA KIT WT. lines and control cells.

An embodiment of the invention is shown below.

### Embodiment 1 (variant 1)

Used in the research were four mastocyte cell lines:
HMC-1.1 containing the pV560G KIT gene mutation;
HMC-1.2 containing the pG560V and pD816V KIT gene mutations,
ROSA KIT D816V containing the pD816V KIT mutation;
ROSA KIT WT - line containing no KIT mutation [21-23].

The HMC lines were obtained from the Department of Allergic Diseases. Mayo Clinic. Rochester. MN, USA

The ROSAKIT lines were obtained from Centre National de la Recherche Scientifique 3 (France), deposited with CNCM (French National Collection of Cultures of Microorganisms) as of 02/11/2011 under numbers: CNCM- 1-4551 ROSA-KIT WT; CNCM-1-4552 ROSA KIT D816V.

### Culture conditions for the HMC line

The culture was grown in a cell incubator at the temperature of 37 °C and at 5% CO2 content, in sterile conditions in a chamber with laminar flow of sterile air. Used was Iscove's Modified Dulbecco's Medium (Merck Millipore) supplemented with 1.2 mM of α-thioglycerol, 50 mL of Foetal Bovine Serum (ATCC), and 5 mL of antibiotics (PEN./STREP. 100x, Merck Millipore)

### Culture conditions for the ROSA KIT line

The culture was grown in a cell incubator at the temperature of 37 °C and at 5% CO2 content. The laboratory works were conducted in sterile conditions in a chamber with laminar flow of sterile air. Used to grow the culture was Iscove's Modified Dulbecco's Medium (Merck Millipore) added with 50 mL of Foetal Bovine Serum (ATCC), and 5 mL of antibiotics (PEN./STREP. 100x, Merck Millipore), while the medium used for the ROSA KIT WT line was additionally supplemented with bone marrow stem cell growth factor rhSCF (R&D Systems, Minneapolis, USA) in the final concentration of 80 ng/ml.

### The procedure

The cells were grown for 48 hours on a 96-well plate at the concentrations 5 ×10^4 cells per well for the HMC 1.1. and HMC 1.2 lines, respectively, and at the concentration of 7.5 ×10^4 cells per well for the ROSA KIT D816V and ROSA KIT WT lines.

Before the experiments were performed, a working solution of 2-methoxyestradiol was produced by dissolving 5 mg of 2-ME (Sigma-Aldrich, USA) in 500 µL of sterile DMSO (dimethyl sulfoxide), so that its final concentration would be 33 mM. Then, the working 2-ME solution was diluted in 100 µL of DMSO so as to obtain the final solution concentrations of 1, 5, and 10 in 100 µL of DMSO, respectively.

The control were cells treated with the corresponding amounts of DMSO with no 2-ME added.

Following 48 hours into incubation, each well was added 50 µL of the XTT solution (Roche Diagnostics GmbH, Mannheim, Germany) prepared in accordance with the manufacturer's instructions. After 4 hours of cell incubation with XTT the level of absorbance was read using the Cytation 3 Imaging Reader (BioTek, USA) at the wavelength of 560 nm.

Application of 2-ME at the concentration of 1 µM on the HMC 1.1 line for 48 hours reduced proliferation of cells by 29%. In the case of the concentration of 5 µM cell viability dropped to 67%, and in the case of 10 µM to 63%.

Following the treatment of 2-ME at the concentration of 1 µM the number of live cells in the HMC 1.2. line dropped to 78%, while at the concentration of 5 µM it dropped by 24%, and by 30% at the concentration of 10 µM.

The ROSA KIT D816V line proved most susceptible to the effects of 2-ME. Following treatment of those cells with 2-ME at the concentration of 1 µM cell viability dropped to 56%. When higher concentrations (5 and 10 µM) were used, the number of live cells dropped to 55% and 51%, respectively.

In the case of the ROSA KIT WT line, 1 µM of 2-ME inhibited the cell growth only by 5%, with 12% recorded for the concentration of 5 µM, and 14% for the concentration of 10 µM.

### Non Patent Literature

Nplcit1: Nedoszytko B., Soko 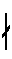 owska-Wojdy 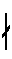 o M., Ruckemann-Dziurdzińska K., Roszkiewicz J., Nowicki RJ. Chemokines and cytokines network in the pathogenesis of the inflammatory skin diseases: Atopic dermatitis, psoriasis and skin mastocytosis. Vol. 31, Postepy Dermatologii i Alergologii. Termedia Publishing House Ltd.; 2014. p. 84-91.

Nplcit2: Broesby-Olsen S., Oropeza AR., Bindslev-Jensen C., Vestergaard H., Møller MB., Siebenhaar F., Kristensen T., Mortz CG. Recognizing mastocytosis in patients with anaphylaxis: Value of KIT D816V mutation analysis of peripheral blood. J Allergy Clin Immunol. 2015;135(1):262-4.

Nplcit3: Carter MC., Bai Y., Ruiz-Esteves KN., Scott LM., Cantave D., Bolan H., Eisch R., Sun X., Hahn J., Maric I., Metcalfe DD. Detection of KIT D816V in peripheral blood of children with manifestations of cutaneous mastocytosis suggests systemic disease. Br J Haematol. 2018;183(5):775-82.

Nplcit4: Renke J., K 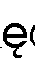 dzierska-Mieszkowska S., Lange M., Nedoszytko B., Wasilewska E., Liberek A., Renke M., Niedoszytko M., Witkowski J., Skórko-Glonek J., Lipińska B. Mast cells in mastocytosis and allergy - Important player in metabolic and immunological homeostasis. Vol. 64, Advances in Medical Sciences. Medical University of Bialystok; 2019. p. 124-30.

Nplcit5: Valent P., Akin C., Bonadonna P., Hartmann K., Brockow K., Niedoszytko M., Nedoszytko B., Siebenhaar F., Sperr WR., Oude Elberink JNG., Butterfield JH., Alvarez-Twose I., Sotlar K., Reiter A., Kluin-Nelemans HC., Hermine O., Gotlib J., Broesby-Olsen S., Orfao A., Horny HP., Triggiani M., Arock M., Schwartz LB., Metcalfe DD. Proposed Diagnostic Algorithm for Patients with Suspected Mast Cell Activation Syndrome. Vol. 7, Journal of Allergy and Clinical Immunology: In Practice. American Academy of Allergy, Asthma and Immunology; 2019. p. 1125-1133.e1.

Nplcit6: Romantowski J., Górska A., Lange M., Nedoszytko B., Gruchata-Niedoszytko M., Niedoszytko M. How to diagnose Mast Cell Activation Syndrome? Practical considerations. Polish Arch Intern Med. 2020;130(4):317-23. 10.20452/pamw.15212

Nplcit7: Matito A., Azaña JM., Torrelo A., Alvarez-Twose I. Cutaneous Mastocytosis in Adults and Children: New Classification and Prognostic Factors. Vol. 38, Immunology and Allergy Clinics of North America. W.B. Saunders; 2018. p. 351-63.

Nplcit8: Parada-Bustamante A., Valencia C., Reuquen P., Diaz P., Rincion-Rodriguez R., Orihuela P. Role of 2-methoxyestradiol, an Endogenous Estrogen Metabolite, in Health and Disease. Mini-Reviews Med Chem. 2015;15(5):427-38. 10.2174/1389557515666150226121052

Nplcit9: Mooberry SL. New insights into 2-methoxyestradiol, a promising antiangiogenic and antitumor agent [Internet]. Vol. 15, Current Opinion in Oncology. 2003. p. 425-30. 10.1097/00001622-200311000-00004

Nplcit10: Harrison MR., Hahn NM., Pili R., Oh WK., Hammers H., Sweeney C., Kim K., Perlman S., Arnott J., Sidor C., Wilding G., Liu G. A phase II study of 2-methoxyestradiol (2ME2) NanoCrystal® dispersion (NCD) in patients with taxane-refractory, metastatic castrate-resistant prostate cancer (CRPC). Invest New Drugs. 2011;29(6):1465-74. 10.1007/s10637-010-9455-x

Nplcit11: LaVallee TM., Burke PA., Swartz GM., Hamel E., Agoston GE., Shah J., Suwandi L., Hanson AD., Fogler WE., Sidor CF., Treston AM. Significant antitumor activity in vivo following treatment with the microtubule agent ENMD-1198. Mol Cancer Ther. 2008;7(6):1472-82.

Nplcit12: Tevaarwerk AJ., Holen KD., Alberti DB., Sidor C., Arnott J., Quon C., Wilding G., Liu G. Phase i trial of 2-methoxyestradiol NanoCrystal dispersion in advanced solid malignancies. Clin Cancer Res. 2009;15(4):1460-5.

Nplcit13: Kumar BS., Raghuvanshi DS., Hasanain M., Alam S., Sarkar J., Mitra K., Khan F., Negi AS. Recent Advances in chemistry and pharmacology of 2-methoxyestradiol: An anticancer investigational drug. Vol. 110, Steroids. Elsevier Inc.; 2016. p. 9-34.

Nplcit14: Mueck AO., Seeger H. 2-methoxyestradiol - Biology and mechanism of action. Vol. 75, Steroids. 2010. p. 625-31.

Nplcit15: Gorska M., Kuban-Jankowska A., Slawek J., Wozniak M. New Insight into 2-Methoxyestradiol- a Possible Physiological Link between Neurodegeneration and Cancer Cell Death. Curr Med Chem. 2016;23(15):1513-27.

Nplcit16: Gorska M., Kuban-Jankowska A., Zmijewski M., Gammazza AM., Cappello F., Wnuk M., Gorzynik M., Rzeszutek I., Daca A., Lewinska A., Wozniak M. DNA strand breaks induced by nuclear hijacking of neuronal NOS as an anticancer effect of 2-methoxyestradiol. Oncotarget. 2015;6(17):15449-63. 10.18632/oncotarget.3913

Nplcit17: Gorska-Ponikowska M., Kuban-Jankowska A., Daca A., Nussberger S. 2-Methoxyestradiol Reverses the Pro-Carcinogenic Effect of L-Lactate in Osteosarcoma 143B Cells. Cancer Genomics Proteomics. 2017;14(6):483-93.

Nplcit18: Gorska M., Kuban-Jankowska A., Milczarek R., Wozniak M. Nitro-oxidative Stress Is Involved in Anticancer Activity of 17beta-Estradiol Derivative in Neuroblastoma Cells. Anticancer Res. 2016;36(4):1693-8.

Nplcit19: Gorska M., Kuban-Jankowska A., Zmijewski M., Gorzynik M., Szkatula M., Wozniak M. Neuronal Nitric Oxide Synthase Induction in the Antitumorigenic and Neurotoxic Effects of 2-Methoxyestradiol. 2014;19(9):13267-81. 10.3390/molecules190913267

Nplcit20: Gorska-Ponikowska M., Perricone U., Kuban-Jankowska A., Lo Bosco G., Barone G. 2-methoxyestradiol impacts on amino acids-mediated metabolic reprogramming in osteosarcoma cells by its interaction with NMDA receptor. J Cell Physiol. 2017;232(11):3030-49.

Nplcit21: NILSSON G., BLOM T., KUSCHE - GULLBERG M., KJELLEN L., BUTTERFIELD JH., SUNDSTROM C., NILSSON K., HELLMAN L. Phenotypic Characterization of the Human Mast - Cell Line HMC - 1. Scand J Immunol. 1994;39(5):489-98.

Nplcit22: Sundström M., Vliagoftis H., Karlberg P., Butterfield JH., Nilsson K., Metcalfe DD., Nilsson G. Functional and phenotypic studies of two variants of a human mast cell line with a distinct set of mutations in the c-kit proto-oncogene. Immunology. 2003;108(1):89-97.

Nplcit23: Furitsu T., Tsujimura T., Tono T., Ikeda H., Kitayama H., Koshimizu U., Sugahara H., Butterfield JH., Ashman LK., Kanayama Y., Matsuzawa Y., Kitamura Y., Kanakura Y. Identification of mutations in the coding sequence of the proto-oncogene c-kit in a human mast cell leukemia cell line causing ligand-independent activation of c-kit product. J Clin Invest. 1993;92(4):1736-44.

## Claims

1. 2-methoxyestradiol for use in the treatment of mastocytosis.

2. 2-methoxyestradiol for use in accordance with Claim 1, where mastocytosis occurs in the skin form and/or systemic form.

3. 2-methoxyestradiol for use in accordance with Claim 1 in the treatment of mastocytosis in children.

4. 2-methoxyestradiol for use in accordance with any of the Claims 1-3 as an agent selectively cytotoxic to neoplastic mastocytes containing a KIT gene mutation.

## Patentansprüche

1. 2-Methoxyestradiol zur Verwendung bei der Behandlung von Mastozytose.

2. 2-Methoxyestradiol zur Verwendung gemäß Anspruch 1, wobei Mastozytose in Hautform und/oder systemischer Form auftritt.

3. 2-Methoxyestradiol zur Verwendung gemäß Anspruch 1 bei der Behandlung von Mastozytose bei Kindern.

4. 2-Methoxyestradiol zur Verwendung gemäß einem der Ansprüche 1-3 als Mittel, das selektiv zytotoxisch für neoplastische Mastozyten ist, die eine KIT-Genmutation enthalten.

## Revendications

1. 2-méthoxyestradiol destiné à être utilisé dans le traitement de mastocytose.

2. 2-méthoxyestradiol destiné à être utilisé selon la revendication 1, dans laquelle la mastocytose survient dans la forme cutanée et/ou systémique.

3. 2-méthoxyestradiol destiné à être utilisé selon la revendication 1 dans le traitement de mastocytose chez l'enfant.

4. 2-méthoxyestradiol destiné à être utilisé selon l'une quelconque des revendications 1-3 en tant qu'un agent sélectivement cytotoxique aux mastocytes néoplasiques présentant une mutation du gène KIT.
